Europäisches Patentamt

European Patent Office

Office européen des brevets

(19)

(11) **EP 1 304 387 A1**

(12) # EUROPEAN PATENT APPLICATION

(43) Date of publication:
**23.04.2003 Bulletin 2003/17**

(51) Int Cl.⁷: **C12Q 1/68**

(21) Application number: **02022398.8**

(22) Date of filing: **10.10.2002**

| | |
|---|---|
| (84) Designated Contracting States:<br>**AT BE BG CH CY CZ DE DK EE ES FI FR GB GR**<br>**IE IT LI LU MC NL PT SE SK TR**<br>Designated Extension States:<br>**AL LT LV MK RO SI** | (72) Inventors:<br>• **Bell, Constance A.**<br>  **Mililani, HI 96789 (US)**<br>• **Uhl, James R.**<br>  **Rochester, MN 55902 (US)**<br>• **Cockerill, Franklin R.**<br>  **Rochester, MN 55905 (US)** |
| (30) Priority: **15.10.2001 US 329826 P**<br>**05.02.2002 US 68238** | |
| | (74) Representative: **Burger, Alexander, Dr.**<br>**Roche Diagnostics GmbH,**<br>**Nonnenwald 2**<br>**82372 Penzberg (DE)** |
| (71) Applicants:<br>• **Roche Diagnostics GmbH**<br>  **68305 Mannheim (DE)**<br>• **Mayo Foundation for Medical Education and**<br>  **Research**<br>  **Rochester, MN 55905-0001 (US)** | |

(54) **Detection of bacillus anthracis**

(57)    The invention provides methods to detect *B. anthracis* in biological or non-biological samples using real-time PCR. Primers and probes for the detection of *B. anthracis* are provided by the invention. Articles of manufacture containing such primers and probes for detecting *B. anthracis* are further provided by the invention.

EP 1 304 387 A1

**Description**

**TECHNICAL FIELD**

This invention relates to bacterial diagnostics, and more particularly to detection of *Bacillus anthracis* (anthrax).

**PRIOR ART BACKGROUND**

Early descriptions of anthrax date to 3,500 years ago; anthrax may have been responsible for two of the plagues that afflicted Egypt in 1491 B.C. In 1877, Koch reported growing the anthrax bacillus *in vitro* and inducing the disease in healthy animals by inoculating them with pure cultures of the bacillus; thus, the model for Koch's famous postulates was born. Around the same time, John Bell recognized woolsorter's disease, or inhalational anthrax; and by setting standards for wool disinfection, he was able to reduce the incidence of this disease in England. William Greenfield and Louis Pasteur were pioneers in anthrax vaccination with their use of a live heat-cured anthrax vaccine for livestock.

**BRIEF DESCRIPTION OF THE INVENTION**

The invention provides for methods of identifying *Bacillus anthracis* in a biological sample or in a non-biological sample. Primers and probes for detecting *B. anthracis* are provided by the invention, as are kits containing such primers and probes. Methods of the invention can be used to rapidly identify *B. anthracis* DNA from specimens for diagnosis of *B. anthracis* infection and to identify hoax cases of *B. anthracis.* Using specific primers and probes, the methods include amplifying and monitoring the development of specific amplification products using fluorescence resonance energy transfer (FRET).

In one aspect of the invention, there is provided a method for detecting the presence or absence of *B. anthracis* in a biological sample from an individual or in a non-biological sample. The method to detect *B. anthracis* includes performing at least one cycling step, which includes an amplifying step and a hybridizing step. The amplifying step includes contacting the sample with a pair of *capB* primers to produce a *capB* amplification product if a *B. anthracis* *capB* nucleic acid molecule is present in the sample. The hybridizing step includes contacting the sample with a pair of *capB* probes. Generally, the members of the pair of *capB* probes hybridize within no more than five nucleotides of each other. A first *capB* probe of the pair of *capB* probes is typically labeled with a donor fluorescent moiety and a second *capB* probe of the pair of *capB* probes is labeled with a corresponding acceptor fluorescent moiety. The method further includes detecting the presence or absence of FRET between the donor fluorescent moiety of the first *capB* probe and the acceptor fluorescent moiety of the second *capB* probe. The presence of FRET is usually indicative of the presence of *B. anthracis* in the sample, while the absence of FRET is usually indicative of the absence of *B. anthracis* in the sample.

Alternatively or additionally, the amplifying step can include contacting the sample with a pair of *pagA* primers to produce a *pagA* amplification product if a *B. anthracis* *pagA* nucleic acid molecule is present in the sample. The hybridizing step includes contacting the sample with a pair of *pagA* probes. Generally, the members of the pair of *pagA* probes hybridize within no more than five nucleotides of each other. A first *pagA* probe of the pair of *pagA* probes is typically labeled with a donor fluorescent moiety and a second *pagA* probe of the pair of *pagA* probes is labeled with a corresponding acceptor fluorescent moiety. The method further includes detecting the presence or absence of FRET between the donor fluorescent moiety of the first *pagA* probe and the acceptor fluorescent moiety of the second *pagA* probe. The presence of FRET is usually indicative of the presence of *B. anthracis* in the sample, while the absence of FRET is usually indicative of the absence of *B. anthracis* in the sample.

Alternatively or additionally, the amplifying step can include contacting the sample with a pair of *lef* primers to produce a *lef* amplification product if a *B. anthracis* *lef* nucleic acid molecule is present in the sample. The hybridizing step includes contacting the sample with a pair of *lef* probes. Generally, the members of the pair of *lef* probes hybridize within no more than five nucleotides of each other. A first *lef* probe of the pair of *lef* probes is typically labeled with a donor fluorescent moiety and a second *lef* probe of the pair of *lef* probes is labeled with a corresponding acceptor fluorescent moiety. The method further includes detecting the presence or absence of FRET between the donor fluorescent moiety of the first *lef* probe and the acceptor fluorescent moiety of the second *lef* probe. The presence of FRET is usually indicative of the presence of *B. anthracis* in the sample, while the absence of FRET is usually indicative of the absence of *B. anthracis* in the sample. The methods to detect *B. anthracis* using *capB, pagA,* and/or *lef* can be performed individually, sequentially or simultaneously.

A pair of *capB* primers generally includes a first *capB* primer and a second *capB* primer. The first *capB* primer can include the sequence 5'-CCC AAT TCG AGT AAA CAT A-3' (SEQ ID NO:1), and the second *capB* primer can include the sequence 5'-ACT GCC ATA CAT TCA CAA-3' (SEQ ID NO:2). A first *capB* probe can include the sequence 5'-CGA TTA AGC GCC GTA AAG AAG GTC CTA ATA TC-3' (SEQ ID NO:3), and the second *capB* probe can include the sequence 5'-GTG AGC AAC GCA GGG TAG TTA AAG AGG CTG-3' (SEQ ID NO:4). A first *capB* probe can alternatively include the sequence 5'-CGC CGT AAA GAA GGT CCT AAT ATC G-3' (SEQ ID NO:13), and a second *capB* probe can alternatively include the sequence 5'-TGA GCA ACG CAG GGT AGT TAA AGA GG-3' (SEQ ID NO: 14).

A pair of *pagA* primers generally includes a first *pagA* primer and a second *pagA* primer. The first *pagA* primer can include the sequence 5'-TAC AGG ACG GAT TGA TAA G-3' (SEQ ID NO:5), and the second *pagA* primer can include the sequence 5'-TTT CAG CCC AAG TTC TTT-3' (SEQ ID NO:6). A first *pagA* probe can include the sequence 5'-AGT ACA TGG AAA TGC AGA AGT G-3' (SEQ ID NO:7) and the second *pagA* probe can include the sequence 5'-ATG CGT CGT TCT TTG ATA TTG GT-3' (SEQ ID NO:8). A first *pagA* probe can alternatively include the sequence 5'-GTG CAT GCG TCG TTC TTT CAT ATT G-3' (SEQ ID NO:15), and a second *pagA* probe can alternatively include the sequence 5'-TGG GAG TGT ATC TGC AGG ATT TAG TAA TTC-3' (SEQ ID NO:16).

A pair of *lef* primers generally includes a first *lef* primer and a second *lef* primer. The first *lef* primer can include the sequence 5'-TTT TAC CGA TAT TAC TCT CC-3' (SEQ ID NO:9), and the second *lef* primer can include the sequence 5'-AAC CTA AGG CTT CTC GC-3' (SEQ ID NO:10). A first *lef* probe can include the sequence 5'-ATT AAG GAA TGA TAG TGA GGG T-3' (SEQ ID NO: 11), and the second *lef* probe can include the sequence 5'-TAT ACA CGA ATT TGG ACA TGC T-3' (SEQ ID NO:12).

In some aspects, one of the *capB, pagA,* or *lef* primers can be labeled with a fluorescent moiety (either a donor or acceptor, as appropriate) and can take the place of the *capB, pagA,* or *lef* probes, respectively.

The members of the pair of *capB, pagA* or *lef* probes can hybridize within no more than two nucleotides of each other, or can hybridize within no more than one nucleotide of each other. A representative donor fluorescent moiety is fluorescein, and corresponding acceptor fluorescent moieties include LC-Red 640, LC-Red 705, Cy5, and Cy5.5. Additional corresponding donor and acceptor fluorescent moieties are known in the art.

In one aspect, the detecting step includes exciting the sample at a wavelength absorbed by the donor fluorescent moiety and visualizing and/or measuring the wavelength emitted by the acceptor fluorescent moiety *(i.e.,* visualizing and'/or measuring FRET). In another aspect, the detecting step includes quantitating the FRET. In yet another aspect, the detecting step can be performed after each cycling step (*e.g.*, in real-time).

Generally, the presence of FRET within 45 cycles (*e.g.*, 20, 25, 30, 35, or 40 cycles) indicates the presence of a *B. anthracis* infection in the individual. In addition, determining the melting temperature between one or both of the *capB* probe(s) and the *capB* amplification product or, similarly, between one or both of the *pagA* or *lef* probe(s) and the *pagA* or *lef* amplification product, respectively, can confirm the presence or absence of the *B. anthracis.*

Representative biological sample include dermal swabs, cerebrospinal fluid, blood, sputum, bronchio-alveolar lavage, bronchial aspirates, lung tissue, and urine. Non-biological samples include powders, air samples, surface swipes, and rinse products from solid materials. Biological or non-biological samples can be cultured. The culture then can be evaluated for *B. anthracis* using the methods of the invention. The above-described methods can further include preventing amplification of a contaminant nucleic acid. Preventing amplification can include performing the amplifying step in the presence of uracil and treating the sample with uracil-DNA glycosylase prior to amplifying.

In addition, the cycling step can be performed on a control sample. A control sample can include the same portion of the *B. anthracis capB* nucleic acid molecule. Alternatively, a control sample can include a nucleic acid molecule other than a *B. anthracis capB* nucleic acid molecule. Cycling steps can be performed on such a control sample using a pair of control primers and a pair of control probes. The control primers and probes are other than *capB* primers and probes. One or more amplifying steps produces a control amplification product. Each of the control probes hybridizes to the control amplification product.

In another aspect of the invention, there are provided articles of manufacture, or kits. Kits of the invention can include a pair of *capB* primers, and a pair of *capB* probes, and a donor and corresponding acceptor fluorescent moieties. For example, the first *capB* primer provided in a kit of the invention can have the sequence 5'-CCC AAT TCG AGT AAA CAT A-3' (SEQ ID NO: 1) and the second *capB* primer can have the sequence 5'-ACT GCC ATA CAT TCA CAA-3' (SEQ ID NO:2). The first *capB* probe provided in a kit of the invention can have the sequence 5'-CGA TTA AGC GCC GTA AAG AAG GTC CTA ATA TC-3' (SEQ ID NO:3) and the second *capB* probe can have the sequence 5'-GTG AGC AAC GCA GGG TAG TTA AAG AGG CTG-3' (SEQ ID NO:4).

Articles of manufacture of the invention can further or alternatively include a pair of *pagA* primers, a pair of *pagA* probes, and a donor and corresponding acceptor fluorescent moieties. For example, the first *pagA* primer provided in a kit of the invention can have the sequence 5'-TAC AGG ACG GAT TGA TAA G-3' (SEQ ID NO:5), and the second *pagA* primer can have the sequence 5'-TTT CAG CCC AAG TTC TTT-3' (SEQ ID NO:6). The first *pagA* probe provided in a kit of the invention can have the sequence 5'-AGT ACA TGG AAA TGC AGA AGT G-3' (SEQ ID NO:7), and the second *pagA* probe can have the sequence 5'-ATG CGT CGT TCT TTG ATA TTG GT-3' (SEQ ID NO:8).

Articles of manufacture of the invention can further or alternatively include a pair of *lef* primers, a pair of *lef* probes, and a donor and corresponding acceptor fluorescent moieties. For example, the first *lef* primer provided in a kit of the invention can have the sequence 5'-TTT TAC CGA TAT TAC TCT CC-3' (SEQ ID NO:9), and the second *lef* primer can have the sequence 5'-AAC CTA AAG GCT TCT GC-3' (SEQ ID NO: 10). The first *lef* probe provided in a kit of the invention can have the sequence 5'-ATT AAG GAA TGA TAG TGA GGG T-3' (SEQ ID NO: 11), and the second *lef* probe can have the sequence 5'-TAT ACA CGA ATT TGG ACA TGC T-3' (SEQ ID NO: 12).

Articles of manufacture can include fluorophoric moieties for labeling the probes or probes already labeled with donor and corresponding acceptor fluorescent moieties. The article of manufacture can also include a package insert having instructions thereon for using the primers, probes, and fluorophoric moieties to detect the presence or absence of *B. anthracis* in a sample.

In yet another aspect of the invention, there is provided a method for detecting the presence or absence of *B. anthracis* in a biological sample from an individual or in a non-biological sample. Such a method includes performing at least one cycling step. A cycling step can include an amplifying step and a hybridizing step. Generally, an amplifying step includes contacting the sample with a pair of *capB* primers to produce a *capB* amplification product if a *B. anthracis capB* nucleic acid molecule is present in the sample. Generally, a hybridizing step includes contacting the sample with a *capB* probe. Such a *capB* probe is usually labeled with a donor fluorescent moiety and a corresponding acceptor fluorescent moiety. The method further includes detecting the presence or absence of fluorescence resonance energy transfer (FRET) between the donor fluorescent moiety and the acceptor fluorescent moiety of the *capB* probe. The presence or absence of fluorescence is indicative of the presence or absence of *B. anthracis* in said sample. In addition to the *capB* primers/probe described herein, this method also can be performed using *pagA* and/or *lef* primers/probe.

In one aspect, amplification can employ a polymerase enzyme having 5' to 3" exonuclease activity. Thus, the first and second fluorescent moieties would be within no more than 5 nucleotides of each other along the length of the probe. In another aspect, the *capB* probe includes a nucleic acid sequence that permits secondary structure formation. Such secondary structure formation generally results in spatial proximity between the first and second fluorescent moiety. According to this method, the second fluorescent moiety on a probe can be a quencher.

In another aspect of the invention, there is provided a method for detecting the presence or absence of *B. anthracis* in a biological sample from an individual or in a non-biological sample. Such a method includes performing at least one cycling step. A cycling step can include an amplifying step and a dye-binding step. An amplifying step generally includes contacting the sample with a pair of *capB* primers to produce a *capB* amplification product if a *B. anthracis capB* nucleic acid molecule is present in the sample. A dye-binding step generally includes contacting the *capB* amplification product with a double-stranded DNA binding dye. The method further includes detecting the presence or absence of binding of the double-stranded DNA binding dye into the amplification product. According to the invention, the presence of binding is typically indicative of the presence of *B. anthracis* in the sample, and the absence of binding is typically indicative of the absence of *B. anthracis* in the sample. Such a method can further include the steps of determining the melting temperature between the *capB* amplification product and the double-stranded DNA binding dye. Generally, the melting temperature confirms the presence or absence of *B. anthracis.* Representative double-stranded DNA binding dyes include SYBRGreenI®, SYBRGold®, and ethidium bromide.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In addition, the materials, methods, and examples are illustrative only and not intended to be limiting. All publications, patent applications, patents, and other references mentioned herein are incorporated by reference in their entirety. In case of conflict, the present specification, including definitions, will control.

The details of one or more embodiments of the invention are set forth in the accompanying drawings and the description below. Other features, objects, and advantages of the invention will be apparent from the drawings and detailed description, and from the claims.

## DETAILED DESCRIPTION

A real-time assay for detecting *B. anthracis* in a biological sample or in a non-biological sample that is more sensitive and specific than existing assays is described herein. Primers and probes for detecting *B. anthracis* infections and articles of manufacture containing such primers and probes are provided by the invention. The increased sensitivity of real-time PCR for detection of *B. anthracis* compared to other methods, as well as the improved features of real-time PCR including sample containment and real-time detection of the amplified product, make feasible the implementation of this technology for routine diagnosis of *B. anthracis* infections in the clinical laboratory.

*Bacillus anthracis*

Anthrax infections are initiated by endospores of *B. anthracis,* a Gram-positive soil organism. Anthrax endospores do not divide, have no measurable metabolism, and are resistant to drying, heat, ultraviolet light, gamma radiation, and many disinfectants. In some types of soil, anthrax spores can remain dormant for decades. All known anthrax virulence genes are expressed by the vegetative form of *B. anthracis* that results from the germination of spores within the body. Endospores introduced into the body by abrasion, inhalation, or ingestion are phagocytosed by macrophages and carried to regional lymph nodes. Endospores germinate inside the macrophages and become vegetative bacteria; the vegetative bacteria are then released from the macrophages, multiply in the lymphatic system, and enter the bloodstream until there are as many as $10^7$ to $10^8$ organisms/ml blood, causing massive septicemia. Once they have been released from the macrophages, there is no evidence that an immune response is initiated against vegetative bacilli. Anthrax bacilli express virulence factors, including toxin and capsule polypeptides. The resulting toxemia has systemic effects that lead to the death of the host.

The major virulence factors of *B. anthracis* are encoded on two virulence plasmids, pX01 and pX02. The toxin-bearing plasmid, pX01, is 184.5 kilobases (Kb) in size and codes for the genes that make up the secreted exotoxins. The toxin gene complex is composed of protective antigen (PA), lethal factor (LF), and edema factor (EF). The three exotoxin components combine to form two binary toxins. Edema toxin consists of EF, which is a calmodulin-dependent adenylate cyclase, and PA, the binding moiety that permits entry of the toxin into the host cell. Increased cellular levels of cyclic AMP upset water homeostasis and are believed to be responsible for the massive edema seen in cutaneous anthrax. Edema toxin inhibits neutrophil function *in vitro* and neutrophil function is impaired in patients with cutaneous anthrax infection. Lethal toxin consists of LF, which is a zinc metalloprotease that inactivates mitogen-activated-protein kinase kinase *in vitro,* and PA, which acts as the binding domain. Lethal toxin stimulates the macrophages to release tumor necrosis factor $^H_J$ and interleukin 1β, which are partly responsible for sudden death in systemic anthrax. The capsule-bearing plasmid, pX02, is 95.3 Kb in size and codes for three genes (*capB, capC, and capA*) involved in the synthesis of the polyglutamyl capsule.

The exotoxins are thought to inhibit the immune response mounted against infection, whereas the capsule inhibits phagocytosis of vegetative anthrax bacilli. The expression of all known major virulence factors is regulated by host-specific factors such as elevated temperature (>37°C) and carbon dioxide concentration (>5%) and by the presence of serum components. Both plasmids are required for full virulence and the loss of either one results in an attenuated strain. Historically, bacterial strains for anthrax vaccines were made by rendering virulent strains free of one or both plasmids. By way of example, 'Pasteur' is an avirulent pX02-carrying strain that is encapsulated but does not express exotoxin components, while 'Sterne' is an attenuated strain that carries pX01 and can synthesize exotoxin components but does not have a capsule.

*A. Genes encoding toxins*

*Protective antigen (PA)*

PA (SwissProt Accession No. P13423; GenBank Accession No. M22589), previously referred to as factor II, is the most extensively characterized component of anthrax toxin. The gene for PA is encoded at the *pag* locus on the plasmid pX01 (formerly known as pBA1). The gene has been cloned and sequenced and found to contain a 2319 basepair (bp) open reading frame of which 2205 bp encode an A/T-rich (69%) cysteine-free, 735 amino acid (83 kilodalton (kDa)) secreted protein. Although PA is a component of anthrax toxin, it is not toxic by itself.

Before PA can translocate EF or LF to the cytosol, PA must first be proteolytically modified. Cleavage occurs at the consensus sequence RKKR, resulting in the removal of a 20-kDa fragment, and yielding PA. Deletions of or mutations at this site render PA resistant to proteolysis and consequently non-toxic in combination with LF or EF. Removal of the NH$_2$-terminal fragment is apparently necessary to expose a region of PA that can bind to the other toxin components, since uncleaved PA can bind to a cell but is unable to associate with EF or LF. Analysis of COOH-terminal truncations or enzymatically-cleaved products of PA indicate that the COOH terminus is necessary for cell binding.

*Lethal factor (LF)*

LF (SwissProt Accession No. P15917; GenBank Accession Nos. M29081 and M30210), previously known as factor III, is encoded on the pX01 plasmid. The gene called *lef* has been cloned and sequenced, and found to contain a 2427 bp open reading frame of which 99 bp encode a 33 amino acid signal peptide and 2328 bp encode an A/T-rich (70%), cysteine-free, 776 amino acid (90.2 kDa) mature, secreted protein. Mitogen-activated protein kinase (MAPK) kinases 1 and 2 (MAPKK1, MAPKK2) act as substrates for LF.

*Edema factor (EF)*

EF (SwissProt Accession No. P40136; GenBank Accession No. M24074), previously referred to as factor 1, is encoded at the *cya* locus on the pX01 plasmid. The gene has been cloned and sequenced and found to contain a 2400-bp-long open reading frame of which 99 bp encode a hydrophobic 33 amino acid signal sequence and 2301 bp encode an A/T-rich (71%) cysteine-free, 767 amino acid (88.8 kDa) mature secreted protein. Sequence analysis of EF indicates that residues 1-250 at the $NH_2$ terminus share homology with the corresponding region of LF. This region of LF has been demonstrated to mediate binding to PA and, when fused to heterologous proteins, is sufficient to allow their translocation to the cytosol via PA.

*B. Routes of Infection*

*Cutaneous anthrax*

Cutaneous anthrax accounts for 95% of all anthrax infections in the U.S. Pathogenic endospores are introduced subcutaneously through a cut or abrasion. There are a few case reports of transmissions by insect bites, presumably after the insect fed on an infected carcass. The primary skin lesion is usually a nondescript, painless, pruritic papule that appears 3 to 5 days after the introduction of endospores. In 24 to 36 hours, the lesion forms a vesicle that undergoes central necrosis and drying, leaving a characteristic black eschar surrounded by edema and a number of purplish vesicles. Histological examination of anthrax skin lesions shows necrosis and massive edema with lymphocytic infiltrates. Gram staining reveals bacilli in the subcutaneous tissue.

*Gastrointestinal and Oropharyngeal anthrax*

The symptoms of gastrointestinal anthrax appear 2 to 5 days after the ingestion of endospore-contaminated meat from diseased animals. It is presumed that bacterial inoculation takes place at a breach in the mucosal lining, but exactly where the endospores germinate is not known. Upon pathological examination, bacilli can be seen microscopically in the mucosal and submucosal lymphatic tissue, and there is gross evidence of mesenteric lymphadenitis. Ulceration is always seen. It is not known whether ulceration occurs only at sites of bacterial infection or is distributed more diffusely as a result of the action of the anthrax toxin. Microscopic examination of affected tissues reveals massive edema and mucosal necrosis at infected sites. Inflammatory infiltrates are seen that are similar to those in cutaneous anthrax. Gram staining of peritoneal fluid may reveal numerous large Gram-positive bacilli. Associated symptoms include fever and diffuse abdominal pain with rebound tenderness. There are reports of both constipation and diarrhea; the stools are either melenic or blood-tinged. Because of ulceration of the gastrointestinal mucosa, patients often vomit material that is blood-tinged or has a coffee ground appearance.
Oropharyngeal anthrax is less common than the gastrointestinal form. It is also associated with the ingestion of contaminated meat. Initial symptoms include cervical edema and local lymphadenopathy, which cause dysphagia and respiratory difficulties. Lesions can be seen in the oropharynx and usually have the appearance of pseudomembranous ulcerations.

*Inhalational anthrax*

When dispersed in the atmosphere as an aerosol, anthrax spores can present a respiratory hazard far downwind from the point of release. Inhalational anthrax is usually fatal, even with aggressive antimicrobial therapy. Anthrax spores are about 1 to 2 μm in diameter, a size that is optimal for inhalation and deposition in the alveolar spaces. Although the lung is the initial site of contact, inhalational anthrax is not considered a true pneumonia. In most but not all cases, there is no infection in the lungs. Rather, the endospores are engulfed by alveolar macrophages and transported by them to the mediastinal and peribronchial lymph nodes, with the spores germinating *en route.* Anthrax bacilli multiply in the lymph nodes, causing hemorrhagic mediastinitis, and spread throughout the body in the blood. Incubation time for inhalational anthrax is approximately 10 days. The onset of symptoms, however, can occur up to six weeks after exposure. Such long incubation times presumably reflect the ability of viable anthrax spores to remain in the lungs for many days. Longer incubation periods may be associated with smaller inocula.
The initial symptoms of inhalational anthrax include fever, nonproductive cough, myalgia, and malaise, resembling those of a viral upper respiratory tract infection. Early in the course of the disease, chest radiographs show a widened mediastinum, which is evidence of hemorrhagic mediastinitis, and marked pleural effusions. After 1 to 3 days, the disease takes a fulminant course with dyspnea, strident cough and chills, culminating in death.

*Anthrax Meningitis*

Involvement of the meninges by *B. anthracis* is a rare complication of anthrax. The most common portal of entry is the skin from which the organisms can spread to the central nervous system by hematogenous or lymphatic routes. Anthrax meningitis also occurs in cases of pulmonary and gastrointestinal anthrax. Anthrax meningitis is almost always fatal, with death occurring 1 to 6 days after the onset of illness, despite intensive antibiotic therapy. In addition to common meningeal symptoms and nuchal rigidity, the patient has fever, fatigue, myalgia, headache, nausea, vomiting, agitation, seizures, and delirium. The initial signs are followed by rapid neurological degeneration and death.

*B. anthracis* nucleic acids and oligonucleotides

The invention provides methods to detect *B. anthracis* by amplifying, for example, a portion of the *B. anthracis capB, pagA,* or *lef* nucleic acid. *B. anthracis* nucleic acids other than those exemplified herein (*e.g.,* other than *capB, pagA,* or *lef*) also can be used to detect *B. anthracis* in a sample and are known to those of skill in the art. The nucleic acid sequence of *B. anthracis capB* (encoding encapsulation protein B), *pagA* (encoding protective antigen), and *lef* (encoding lethal factor) are available (see, for example, GenBank Accession Nos. M24150, M22589 and M30210, respectively). Specifically, primers and probes to amplify and detect *B. anthracis capB* nucleic acid molecules are provided by the invention as are primers and probes to amplify and detect *B. anthracis pagA* nucleic acid molecules and *B. anthracis lef* nucleic acid molecules.

Primers that amplify a *B. anthracis* nucleic acid molecule, *e.g., B. anthracis capB, pagA,* or *lef,* can be designed using, for example, a computer program such as OLIGO (Molecular Biology Insights, Inc., Cascade, CO). Important features when designing oligonucleotides to be used as amplification primers include, but are not limited to, an appropriate size amplification product to facilitate detection (*e.g.,* by electrophoresis), similar melting temperatures for the members of a pair of primers, and the length of each primer (*i.e.*, the primers need to be long enough to anneal with sequence-specificity and to initiate synthesis but not so long that fidelity is reduced during oligonucleotide synthesis). Typically, oligonucleotide primers are 15 to 30 nucleotides in length.

Designing oligonucleotides to be used as hybridization probes can be performed in a manner similar to the design of primers, although the members of a pair of probes preferably anneal to an amplification product within no more than 5 nucleotides of each other on the same strand such that FRET can occur (*e.g.,* within no more than 1, 2, 3, or 4 nucleotides of each other). This minimal degree of separation typically brings the respective fluorescent moieties into sufficient proximity such that FRET occurs. It is to be understood, however, that other separation distances (*e.g.,* 6 or more nucleotides) are possible provided the fluorescent moieties are appropriately positioned relative to each other (for example, with a linker arm) such that FRET can occur. In addition, probes can be designed to hybridize to targets that contain a polymorphism or mutation, thereby allowing differential detection of *B. anthracis* strains based on either absolute hybridization of different pairs of probes corresponding to the particular *B. anthracis* strain to be distinguished or differential melting temperatures between, for example, members of a pair of probes and each amplification product corresponding to a *B. anthracis* strain to be distinguished. As with oligonucleotide primers, oligonucleotide probes usually have similar melting temperatures, and the length of each probe must be sufficient for sequence-specific hybridization to occur but not so long that fidelity is reduced during synthesis. Oligonucleotide probes are generally 15 to 30 nucleotides in length.

Constructs of the invention include vectors containing a *B. anthracis* nucleic acid molecule, *e.g., B. anthracis capB, pagA,* or *lef,* or a fragment thereof. Constructs of the invention can be used, for example, as control template nucleic acid molecules. Vectors suitable for use in the present invention are commercially available and/or produced by recombinant DNA technology methods routine in the art. *B. anthracis capB, pagA* or *lef* nucleic acid molecules can be obtained, for example, by chemical synthesis, direct cloning from *B. anthracis*, or by PCR amplification. A *B. anthracis* nucleic acid molecule or fragment thereof can be operably linked to a promoter or other regulatory element such as an enhancer sequence, a response element, or an inducible element that modulates expression of the *B. anthracis* nucleic acid molecule. As used herein, operably linking refers to connecting a promoter and/or other regulatory elements to a *B. anthracis* nucleic acid molecule in such a way as to permit and/or regulate expression of the *B. anthracis* nucleic acid molecule. A promoter that does not normally direct expression of *B. anthracis capB, pagA* or *lef* can be used to direct transcription of a *capB, pagA,* or *lef* nucleic acid using, for example, a viral polymerase, a bacterial polymerase, or a eukaryotic RNA polymerase II. Alternatively, the *capB, pagA* or *lef* native promoter can be used to direct transcription of a *capB, pagA,* or *lef* nucleic acid, respectively, using, for example, a *B. anthracis* RNA polymerase enzyme. In addition, operably linked can refer to an appropriate connection between a *B. anthracis capB, pagA,* or *lef* promoter or regulatory element and a heterologous coding sequence (*i.e.,* a non-*capB, -pagA,* or -*lef* coding sequence, for example, a reporter gene) in such a way as to permit expression of the heterologous coding sequence.

Constructs suitable for use in the methods of the invention typically include, in addition to *B. anthracis capB, pagA* or *lef* nucleic acid molecules, sequences encoding a selectable marker (*e.g,* an antibiotic resistance gene) for selecting desired constructs and/or transformants, and an origin of replication. The choice of vector systems usually depends upon several factors, including, but not limited to, the choice of host cells, replication efficiency, selectability, inducibility, and the ease of recovery.

Constructs of the invention containing *B. anthracis capB, pagA,* or *lef* nucleic acid molecules can be propagated in a host cell. As used herein, the term host cell is meant to include prokaryotes and eukaryotes such as yeast, plant and animal cells. Prokaryotic hosts may include E. *coli, Salmonella typhimurium, Serratia marcescens* and *Bacillus subtilis.* Eukaryotic hosts include yeasts such as *S. cerevisiae, S. pombe, Pichia pastoris*, mammalian cells such as COS cells or Chinese hamster ovary (CHO) cells, insect cells, and plant cells such as *Arabidopsis thaliana* and *Nicotiana tabacum.* A construct of the invention can be introduced into a host cell using any of the techniques commonly known to those of ordinary skill in the art. For example, calcium phosphate precipitation, electroporation, heat shock, lipofection, microinjection, and viral-mediated nucleic acid transfer are common methods for introducing nucleic acids into host cells. In addition, naked DNA can be delivered directly to cells (see, *e.g.,* U.S. Patent Nos. 5,580,859 and 5,589,466).

Polymerase chain reaction (PCR)

U.S. Patent Nos. 4,683,202, 4,683,195, 4,800,159, and 4,965,188 disclose conventional PCR techniques. PCR typically employs two oligonucleotide primers that bind to a selected nucleic acid template (*e.g.,* DNA or RNA). Primers useful in the present invention include oligonucleotides capable of acting as a point of initiation of nucleic acid synthesis within *B. anthracis capB, pagA,* or *lef.* A primer can be purified from a restriction digest by conventional methods, or it can be produced synthetically. The primer is preferably single-stranded for maximum efficiency in amplification, but the primer can be double-stranded. Double-stranded primers are first denatured, *i.e.,* treated to separate the strands. One method of denaturing double stranded nucleic acids is by heating.

The term "thermostable polymerase" refers to a polymerase enzyme that is heat stable, *i.e.,* the enzyme catalyzes the formation of primer extension products complementary to a template and does not irreversibly denature when subjected to the elevated temperatures for the time necessary to effect denaturation of double-stranded template nucleic acids. Generally, the synthesis is initiated at the 3' end of each primer and proceeds in the 5' to 3' direction along the template strand. Thermostable polymerases have been isolated from *Thermus flavus, T. ruber*, *T. thermophilus, T. aquaticus, T. lacteus, T. rubens, Bacillus stearothermophilus,* and *Methanothermus fervidus*. Nonetheless, polymerases that are not thermostable also can be employed in PCR assays provided the enzyme is replenished.

If the *B. anthracis* template nucleic acid is double-stranded, it is necessary to separate the two strands before it can be used as a template in PCR. Strand separation can be accomplished by any suitable denaturing method including physical, chemical or enzymatic means. One method of separating the nucleic acid strands involves heating the nucleic acid until it is predominately denatured (*e.g.*, greater than 50%, 60%, 70%, 80%, 90% or 95% denatured). The heating conditions necessary for denaturing template nucleic acid will depend, *e.g.,* on the buffer salt concentration and the length and nucleotide composition of the nucleic acids being denatured, but typically range from about 90°C to about 105°C for a time depending on features of the reaction such as temperature and the nucleic acid length. Denaturation is typically performed for about 30 sec to 4 min.

If the double-stranded nucleic acid is denatured by heat, the reaction mixture is allowed to cool to a temperature that promotes annealing of each primer to its target sequence on the *B. anthracis* nucleic acid. The temperature for annealing is usually from about 35°C to about 65°C. Annealing times can be from about 10 secs to about 1 min. The reaction mixture is then adjusted to a temperature at which the activity of the polymerase is promoted or optimized, *i.e.*, a temperature sufficient for extension to occur from the annealed primer to generate products complementary to the template nucleic acid. The temperature should be sufficient to synthesize an extension product from each primer that is annealed to a nucleic acid template, but should not be so high as to denature an extension product from its complementary template (*e.g.,* the temperature for extension generally ranges from about 40° to 80°C). Extension times can be from about 10 secs to about 5 mins.

PCR assays can employ *B. anthracis* nucleic acid such as DNA or RNA, including messenger RNA (mRNA). The template nucleic acid need not be purified; it may be a minor fraction of a complex mixture, such as *B. anthracis* nucleic acid contained in human cells. DNA or RNA may be extracted from a biological sample such as dermal swabs, cerebrospinal fluid, blood, sputum, bronchio-alveolar lavage, bronchial aspirates, lung tissue, and feces by routine techniques such as those described in *Diagnostic Molecular Microbiology: Principles and Applications* (Persing et al. (eds), 1993, American Society for Microbiology, Washington D.C). If *B. anthracis* is present, DNA or RNA also can be extracted from non-biological samples such as air samples, suspicious powders, surface swipes and rinse products from suspicious solid materials. Nucleic acids can be obtained from any number of sources, such as plasmids, or natural sources including bacteria, yeast, viruses, organelles, or higher organisms such as plants or animals.

The oligonucleotide primers are combined with PCR reagents under reaction conditions that induce primer extension. For example, chain extension reactions generally include 50 mM KC1, 10 mM Tris-HCl (pH 8.3), 15 mM MgCl$_2$, 0.001% (w/v) gelatin, 0.5-1.0 $^c$cg denatured template DNA, 50 pmoles of each oligonucleotide primer, 2.5 U of Taq polymerase, and 10% DMSO). The reactions usually contain 150 to 320 $^c_c$M each of dATP, dCTP, dTTP, dGTP, or one or more analogs thereof.

The newly synthesized strands form a double-stranded molecule that can be used in the succeeding steps of the reaction. The steps of strand separation, annealing, and elongation can be repeated as often as needed to produce the desired quantity of amplification products corresponding to the target *B. anthracis* nucleic acid molecule. The limiting factors in the reaction are the amounts of primers, thermostable enzyme, and nucleoside triphosphates present in the reaction. The cycling steps (*i.e.*, denaturation, annealing, and extension) are preferably repeated at least once. For use in detection, the number of cycling steps will depend, *e.g.*, on the nature of the sample. If the sample is a complex mixture of nucleic acids, more cycling steps will be required to amplify the target sequence sufficient for detection. Generally, the cycling steps are repeated at least about 20 times, but may be repeated as many as 40, 60, or even 100 times.

Fluorescence resonance energy transfer (FRET)

FRET technology (see, for example, U.S. Patent Nos. 4,996,143, 5,565,322, 5,849,489, and 6,162,603) is based on a concept that when a donor and a corresponding acceptor fluorescent moiety are positioned within a certain distance of each other, energy transfer takes place between the two fluorescent moieties that can be visualized or otherwise detected and/or quantitated. Two oligonucleotide probes, each containing a fluorescent moiety, can hybridize to an amplification product at particular positions determined by the complementarity of the oligonucleotide probes to the *B. anthracis* target nucleic acid sequence. Upon hybridization of the oligonucleotide probes to the amplification product nucleic acid at the appropriate positions, a FRET signal is generated. Hybridization temperatures can range from about 35 to about 65°C for about 10 secs to about 1 min.

Fluorescent analysis can be carried out using, for example, a photon counting epifluorescent microscope system (containing the appropriate dichroic mirror and filters for monitoring fluorescent emission at the particular range), a photon counting photomultiplier system or a fluorometer. Excitation to initiate energy transfer can be carried out with an argon ion laser, a high intensity mercury (Hg) arc lamp, a fiber optic light source, or other high intensity light source appropriately filtered for excitation in the desired range.

As used herein with respect to donor and corresponding acceptor fluorescent moieties "corresponding" refers to an acceptor fluorescent moiety having an emission spectrum that overlaps the excitation spectrum of the donor fluorescent moiety. The wavelength maximum of the emission spectrum of the acceptor fluorescent moiety should be at least 100 nm greater than the wavelength maximum of the excitation spectrum of the donor fluorescent moiety. Accordingly, efficient non-radiative energy transfer can be produced therebetween.

Fluorescent donor and corresponding acceptor moieties are generally chosen for (a) high efficiency Förster energy transfer; (b) a large final Stokes shift (>100 nm); (c) shift of the emission as far as possible into the red portion of the visible spectrum (>600 nm); and (d) shift of the emission to a higher wavelength than the Raman water fluorescent emission produced by excitation at the donor excitation wavelength. For example, a donor fluorescent moiety can be chosen that has its excitation maximum near a laser line (for example, Helium-Cadmium 442 nm or Argon 488 nm), a high extinction coefficient, a high quantum yield, and a good overlap of its fluorescent emission with the excitation spectrum of the corresponding acceptor fluorescent moiety. A corresponding acceptor fluorescent moiety can be chosen that has a high extinction coefficient, a high quantum yield, a good overlap of its excitation with the emission of the donor fluorescent moiety, and emission in the red part of the visible spectrum (>600 nm).

Representative donor fluorescent moieties that can be used with various acceptor fluorescent moieties in FRET technology include fluorescein, Lucifer Yellow, B-phycoerythrin, 9-acridineisothiocyanate, Lucifer Yellow VS, 4-acetamido-4'-isothiocyanatostilbene-2,2'-disulfonic acid, 7-diethylamino-3-(4'-isothiocyanatophenyl)-4-methylcoumarin, succinimdyl 1-pyrenebutyrate, and 4-acetamido-4'-isothiocyanatostilbene-2,2'-disulfonic acid derivatives. Representative acceptor fluorescent moieties, depending upon the donor fluorescent moiety used, include LC™-Red 640, LC™-Red 705, Cy5, Cy5.5, Lissamine rhodamine B sulfonyl chloride, tetramethyl rhodamine isothiocyanate, rhodamine x isothiocyanate, erythrosine isothiocyanate, fluorescein, diethylenetriamine pentaacetate or other chelates of Lanthanide ions (*e.g.*, Europium, or Terbium). Donor and acceptor fluorescent moieties can be obtained, for example, from Molecular Probes (Junction City, OR) or Sigma Chemical Co. (St. Louis, MO).

The donor and acceptor fluorescent moieties can be attached to the appropriate probe oligonucleotide via a linker arm. The length of each linker arm is important, as the linker arms will affect the distance between the donor and acceptor fluorescent moieties. The length of a linker arm for the purpose of the present invention is the distance in Angstroms (Å) from the nucleotide base to the fluorescent moiety. In general, a linker arm is from about 10 to about 25 Å. The linker arm may be of the kind described in WO 84/03285. WO 84/03285 also discloses methods for attaching linker arms to a particular nucleotide base, and also for attaching fluorescent moieties to a linker arm.

An acceptor fluorescent moiety such as an LC™-Red 640-NHS-ester can be combined with C6-Phosphoramidites (available from ABI (Foster City, CA) or Glen Research (Sterling, VA)) to produce, for example, LC™-Red 640-Phosphoramidite. Frequently used linkers to couple a donor fluorescent moiety such as fluorescein to an oligonucleotide include thiourea linkers (FITC-derived, for example, fluorescein-CPG's from Glen Research or ChemGene (Ashland, MA)), amide-linkers (fluorescein-NHS-ester-derived, such as fluorescein-CPG from BioGenex (San Ramon, CA)), or 3'-amino-CPG's that require coupling of a fluorescein-NHS-ester after oligonucleotide synthesis.

Detection of *B. anthracis*

The presence of *B. anthracis* has been detected by culturing the organism. The success of culturing *B. anthracis* from clinical specimens depends in part upon the *B. anthracis* infection and hence, the source of the specimen. Cultures from skin lesions associated with the cutaneous form of the disease can exhibit 60-65% sensitivity, and often are not diagnostically useful. Generally, cultures from blood exhibit a high sensitivity due to the extremely large number of circulating *B. anthracis* organisms. Patients with systemic disease, however, often expire within the time necessary for blood cultures to become positive. Other biological samples including sputum and cerebrospinal fluid (CSF) can also be cultured, but the identification may come too late to initiate effective antibiotic therapy. In the case of gastrointestinal anthrax, cultures of stool samples also can be used.

Serologic tests including enzyme-linked immunosorbent assays (ELISAs) and indirect microhemagglutination for the detection of *B. anthracis* have been reported. The sensitivity of ELISA to detect serum antibodies to various targets in *B. anthracis* including the encapsulation protein, protective antigen, lethal factor, and edema factors varies between 26% and 100%, depending upon the target and study. Microagglutination assays also can be used, but such assays are technically demanding for routine clinical laboratory diagnostics. These serologic tests can be used for retrospective study and possibly for cutaneous cases of *B. anthracis* in which the infection does not progress too quickly. However, serological tests generally are not used in cases of acute illness such as would be seen with inhalation anthrax.

Direct detection of *B. anthracis* from clinical specimens or suspicious substances using stains is possible and can allow a presumptive identification of *B. anthracis.* With Gram staining, the spores are visualized as large Gram-positive encapsulated rods. India ink is also useful for the visualization of encapsulated *B. anthracis* organisms, with the capsule appearing as a well-defined cleat zone around the organisms. The success of these staining techniques depends upon the presence of a sufficient number of organisms. Typically, staining techniques provide a presumptive identification of *B. anthracis* and a definitive diagnosis generally requires further evaluation.

The anthraxin skin test can be performed to detect *B. anthracis.* The anthraxin skin test consists of a subdermal injection of a commercially produced chemical extract of an attenuated strain of *B. anthracis.* The sensitivity of skin testing depends upon the cell-mediated immunity being mounted by the patient, and the use of skin testing in rapidly progressing acute disease can be limited.

Conventional PCR methods also have been used to detect *B. anthracis. B. anthracis* and other members of the *B. cereus* group, however, exhibit a high degree of genomic homology, making detection and differentiation by PCR difficult. Conventional PCR-based amplification is generally followed by transfer of the amplification products to a solid support and detection using a labeled probe (*e.g.,* a Southern or Northern blot). These methods are labor intensive and frequently require more than one day to complete. Additionally, the manipulation of amplification products for the purpose of detection (*e.g.,* by blotting) increases the risk of carry-over contamination and false positives. By using commercially available real-time PCR instrumentation (*e.g.,* LightCycler™, Roche Molecular Biochemicals, Indianapolis, IN), PCR amplification and detection of the amplification product can be combined in a single closed cuvette with dramatically reduced cycling time. Since detection occurs concurrently with amplification, the real-time PCR methods obviate the need for manipulation of the amplification product, and diminish the risk of cross-contamination between amplification products. Real-time PCR greatly reduces turn-around time and is an attractive alternative to conventional PCR techniques in the clinical laboratory.

The present invention provides methods for detecting the presence or absence of *B. anthracis* in a biological sample from an individual or in a non-biological sample. Methods provided by the invention avoid problems of sample contamination, false negatives, and false positives. The methods include performing at least one cycling step that includes amplifying a *B. anthracis* portion of a *capB, pagA,* and/or *lef* nucleic acid molecule from a sample using a pair of *capB, pagA,* and/or *lef* primers, respectively. Each of the *capB, pagA,* or *lef* primers anneals to a target within or adjacent to a *B. anthracis capB, pagA,* or *lef* nucleic acid molecule, respectively, such that at least a portion of each amplification product contains nucleic acid sequence corresponding to *capB*, *pagA*, or *lef*, respectively. More importantly, the amplification product should contain the nucleic acid sequences that are complementary to the *capB, pagA,* or *lef* probes, respectively. The *capB, pagA,* and/or *lef* amplification product is produced provided that *B. anthracis* nucleic acid is present. Each cycling step further includes contacting the sample with a pair of *capB, pagA,* and/or *lef* probes. According to the invention, one member of each pair of the *capB, pagA,* and *lef* probes is labeled with a donor fluorescent moiety and the other is labeled with a corresponding acceptor fluorescent moiety. The presence or absence of FRET between the donor fluorescent moiety of the first *capB, pagA,* or *lef* probe and the corresponding acceptor fluorescent moiety of the second *capB, pagA,* or *lef* probe, respectively, is detected upon hybridization of the *capB, pagA,* or *lef* probes to the respective amplification product.

Each cycling step includes an amplification step and a hybridization step, and each cycling step is usually followed by a FRET detecting step. Multiple cycling steps are performed, preferably in a thermocycler. Methods of the invention can be performed using one or more of the *capB*, *pagA* and/or *lef* primer and probe sets to detect the presence of *B. anthracis.* Alternatively, methods of the invention can be performed simultaneously with each of the *capB, pagA* and *lef* primer and probe sets to detect the presence of virulent forms of *B. anthracis.* Detection of FRET in one or more, but not all, of the *capB, pagA* and *lef* reactions indicates the presence of a *B. anthracis* strain lacking one or both of the virulence plasmids. Methods of the invention, therefore, also are useful for detecting false or hoax claims of anthrax.

As used herein, "amplifying" refers to the process of synthesizing nucleic acid molecules that are complementary to one or both strands of a template nucleic acid molecule (*e.g.*, *B. anthracis capB*, *pagA,* or *lef* nucleic acid molecules). Amplifying a nucleic acid molecule typically includes denaturing the template nucleic acid, annealing primers to the template nucleic acid at a temperature that is below the melting temperatures of the primers, and enzymatically elongating from the primers to generate an amplification product. Amplification typically requires the presence of deoxyribonucleoside triphosphates, a DNA polymerase enzyme (*e.g.*, Platinum® Taq) and an appropriate buffer and/or co-factors for optimal activity of the polymerase enzyme (*e.g.*, MgCl$_2$ and/or KCl).

If amplification of *B. anthracis* nucleic acid occurs and an amplification product is produced, the step of hybridizing results in a detectable signal based upon FRET between the members of the pair of probes. As used herein, "hybridizing" refers to the annealing of probes to an amplification product. Hybridization conditions typically include a temperature that is below the melting temperature of the probes but that avoids nonspecific hybridization of the probes.

Generally, the presence of FRET indicates the presence of *B. anthracis* in the sample, and the absence of FRET indicates the absence of *B. anthracis* in the sample. Inadequate specimen collection, transportation delays, inappropriate transportation conditions, or use of certain collection swabs (calcium alginate or aluminum shaft) are all conditions that can affect the success and/or accuracy of a test result, however. Using the methods disclosed herein, detection of FRET within 45 cycling steps is indicative of a *B. anthracis* infection.

Methods of the invention also can be used for *B. anthracis* vaccine efficacy studies or epidemiology studies. For example, an attenuated *B. anthracis* in an anthrax vaccine can be detected using the methods of the invention during the time when bacteria is still present in an individual. For such vaccine efficacy studies, the methods of the invention can be used to determine, for example, the persistence of an attenuated strain of *B. anthracis* used in a vaccine, or can be performed in conjunction with an additional assay such as a serologic assay to monitor an individual's immune response to such a vaccine. In addition, methods of the invention can be used to distinguish one *B. anthracis* strain from another for epidemiology studies of, for example, the origin or severity of an outbreak of *B. anthracis.*

Representative biological samples that can be used in practicing the methods of the invention include dermal swabs, cerebrospinal fluid, blood, sputum, bronchio-alveolar lavage, bronchial aspirates, lung tissue, and feces. Collection and storage methods of biological samples are known to those of skill in the art. Biological samples can be processed (*e.g.,* by nucleic acid extraction methods and/or kits known in the art) to release *B. anthracis* nucleic acid or in some cases, the biological sample can be contacted directly with the PCR reaction components and the appropriate oligonucleotides.

Non-biological samples such as air samples (filtered or non-filtered), powders, and surface swipes and rinse products from suspicious materials also can be examined for the detection of *B. anthracis.* For example, a powder can be dissolved in a solvent such as water, and the methods of the invention can be performed on varying dilutions (*e.g.,* 1:10, 1:100, or 1:1000) of the resulting solution. Water can be added to a collection vial of an air sample collection device and assayed using methods of the invention, or alternatively, a filter on an air sample collection device can be rinsed and assayed. In addition, a solid material (*e.g.,* paper) can be swiped or rinsed for the purpose of detecting *B. anthracis,* and a non-turbid solution produced. Dilutions of such a surface swipe or rinse can be used in a real-time amplification reaction of the invention.

Biological or non-biological samples can be cultured in a medium suitable for growth of *B. anthracis.* The culture media then can be assayed for the presence or absence of *B. anthracis* using the methods of the invention as described herein. For example, samples arriving at a clinical laboratory for detection of *B. anthracis* using the methods of the invention can be in the form of a liquid culture that had been inoculated with a biological sample from an individual or with a non-biological sample.

Melting curve analysis is an additional step that can be included in a cycling profile. Melting curve analysis is based on the fact that DNA melts at a characteristic temperature called the melting temperature (Tm), which is defined as the temperature at which half of the DNA duplexes have separated into single strands. The melting temperature of a DNA depends primarily upon its nucleotide composition. Thus, DNA molecules rich in G and C nucleotides have a higher Tm than those having an abundance of A and T nucleotides. By detecting the temperature at which signal is lost, the melting temperature of probes can be determined. Similarly, by detecting the temperature at which signal is generated, the annealing temperature of probes can be determined. The melting temperature(s) of the *capB, pagA,* or *lef* probes from the respective amplification product can confirm the presence or absence of *B. anthracis* in the sample.

Within each thermocycler run, control samples are cycled as well. Positive control samples can amplify *B. anthracis* nucleic acid control template (other than *capB, pagA,* or *lef*) using, for example, control primers and control probes. Positive control samples can also amplify, for example, a plasmid construct containing *B. anthracis capB, pagA,* or *lef* nucleic acid molecule. Such a plasmid control can be amplified internally (*e.g.,* within the sample) or in a separate sample run side-by-side with the patients' samples. Each thermocycler run should also include a negative control that, for example, lacks *B. anthracis* template DNA. Such controls are indicators of the success or failure of the amplification, hybridization and/or FRET reaction. Therefore, control reactions can readily determine, for example, the ability of primers to anneal with sequence-specificity and to initiate elongation, as well as the ability of probes to hybridize with sequence-specificity and for FRET to occur.

In an embodiment, the methods of the invention include steps to avoid contamination. For example, an enzymatic method utilizing uracil-DNA glycosylase is described in U.S. Patent Nos. 5,035,996, 5,683,896 and 5,945,313 to reduce or eliminate contamination between one thermocycler run and the next. In addition, standard laboratory containment practices and procedures are desirable when performing methods of the invention. Containment practices and procedures include, but are not limited to, separate work areas for different steps of a method, containment hoods, barrier filter pipette tips and dedicated air displacement pipettes. Consistent containment practices and procedures by personnel are necessary for accuracy in a diagnostic laboratory handling clinical samples.

Conventional PCR methods in conjunction with FRET technology can be used to practice the methods of the invention. In one embodiment, a LightCycler™ instrument is used. A detailed description of the LightCycler™ System and real-time and on-line monitoring of PCR can be found at http://biochem.roche.com/lightcycler. The following patent applications describe real-time PCR as used in the LightCycler™ technology: WO 97/46707, WO 97/46714 and WO 97/46712. The LightCycler™ instrument is a rapid thermal cycler combined with a microvolume fluorometer utilizing high quality optics. This rapid thermocycling technique uses thin glass cuvettes as reaction vessels. Heating and cooling of the reaction chamber are controlled by alternating heated and ambient air. Due to the low mass of air and the high ratio of surface area to volume of the cuvettes, very rapid temperature exchange rates can be achieved within the LightCycler™ thermal chamber. Addition of selected fluorescent dyes to the reaction components allows the PCR to be monitored in real time and on-line. Furthermore, the cuvettes serve as an optical element for signal collection (similar to glass fiber optics), concentrating the signal at the tip of the cuvette. The effect is efficient illumination and fluorescent monitoring of microvolume samples.

The Lightcycler™ carousel that houses the cuvettes can be removed from the instrument. Therefore, samples can be loaded outside of the instrument (in a PCR Clean Room, for example). In addition, this feature allows for the sample carousel to be easily cleaned and sterilized. The fluorometer, as part of the LightCycler™ apparatus, houses the light source. The emitted light is filtered and focused by an epi-illumination lens onto the top of the cuvette. Fluorescent light emitted from the sample is then focused by the same lens, passed through a dichroic mirror, filtered appropriately, and focused onto data-collecting photohybrids. The optical unit currently available in the LightCycler™ instrument (Roche Molecular Biochemicals, Catalog No. 2 011 468) includes three bandpass filters (530 nm, 640 nm, and 710 nm), providing three-color detection and several fluorescence acquisition options. Data collection options include once per cycling step monitoring, fully continuous single-sample acquisition for melting curve analysis, continuous sampling (in which sampling frequency is dependent on sample number) and/or stepwise measurement of all samples after defined temperature interval.

The LightCycler™ can be operated using a PC workstation and can utilize a Windows NT operating system. Signals from the samples are obtained as the machine positions the capillaries sequentially over the optical unit. The software can display the fluorescence signals in real-time immediately after each measurement. Fluorescent acquisition time is 10-100 milliseconds (msec). After each cycling step, a quantitative display of fluorescence vs. cycle number can be continually updated for all samples. The data generated can be stored for further analysis.

As an alternative to FRET, an amplification product can be detected using a double-stranded DNA binding dye such as a fluorescent DNA binding dye (*e.g.,* SYBRGreenl® or SYBRGold® (Molecular Probes)). Upon interaction with the double-stranded nucleic acid, such fluorescent DNA binding dyes emit a fluorescence signal after excitation with light at a suitable wavelength. A double-stranded DNA binding dye such as a nucleic acid intercalating dye also can be used. When double-stranded DNA binding dyes are used, a melting curve analysis is usually performed for confirmation of the presence of the amplification product.

As described herein, amplification products also can be detected using labeled hybridization probes that take advantage of FRET technology. A common format of FRET technology utilizes two hybridization probes. Each probe can be labeled with a different fluorescent moiety and are generally designed to hybridize in close proximity to each other in a target DNA molecule (*e.g.,* an amplification product). A donor fluorescent moiety, for example, fluorescein, is excited at 470 nm by the light source of the LightCycler™ Instrument. During FRET, the fluorescein transfers its energy to an acceptor fluorescent moiety such as LightCycler™-Red 640 (LC™-Red 640) or LightCycler™-Red 705 (LC™-Red 705). The acceptor fluorescent moiety then emits light of a longer wavelength, which is detected by the optical detection system of the LightCycler™ instrument. Efficient FRET can only take place when the fluorescent moieties are in direct local proximity and when the emission spectrum of the donor fluorescent moiety overlaps with the absorption spectrum of the acceptor fluorescent moiety. The intensity of the emitted signal can be correlated with the number of original target DNA molecules (*e.g.,* the number of *B. anthracis* genomes).

Another FRET format utilizes TaqMan® technology to detect the presence or absence of an amplification product, and hence, the presence or absence of *B. anthracis.* TaqMan® technology utilizes one single-stranded hybridization probe labeled with two fluorescent moieties. When a first fluorescent moiety is excited with light of a suitable wavelength, the absorbed energy is transferred to a second fluorescent moiety according to the principles of FRET. The second fluorescent moiety is generally a quencher molecule. During the annealing step of the PCR reaction, the labeled hybridization probe binds to the target DNA (*i.e.,* the amplification product) and is degraded by the 5' to 3' exonuclease activity of the Taq Polymerase during the subsequent elongation phase. As a result, the excited fluorescent moiety and the quencher moiety become spatially separated from one another. As a consequence, upon excitation of the first fluorescent moiety in the absence of the quencher, the fluorescence emission from the first fluorescent moiety can be detected. By way of example, an ABI PRISM® 7700 Sequence Detection System (Applied Biosystems, Foster City, CA) uses TaqMan® technology, and is suitable for performing the methods described herein for detecting *B. anthracis.* Information on PCR amplification and detection using an ABI PRISM® 770 system can be found at http://www.appliedbiosystems.com/products.

Molecular beacons in conjunction with FRET also can be used to detect the presence of an amplification product using the real-time PCR methods of the invention. Molecular beacon technology uses a hybridization probe labeled with a first fluorescent moiety and a second fluorescent moiety. The second fluorescent moiety is generally a quencher, and the fluorescent labels are typically located at each end of the probe. Molecular beacon technology uses a probe oligonucleotide having sequences that permit secondary structure formation (*e.g.,* a hairpin). As a result of secondary structure formation within the probe, both fluorescent moieties are in spatial proximity when the probe is in solution. After hybridization to the target nucleic acids (*i.e.,* amplification products), the secondary structure of the probe is disrupted and the fluorescent moieties become separated from one another such that after excitation with light of a suitable wavelength, the emission of the first fluorescent moiety can be detected.

It is understood that the present invention is not limited by the configuration of one or more commercially available instruments.

Articles of manufacture

The invention further provides for articles of manufacture to detect *B. anthracis.* An article of manufacture according to the present invention can include primers and probes used to detect *B. anthracis,* together with suitable packaging materials. Representative primers and probes for detection of *B. anthracis* are capable of hybridizing to *B. anthracis capB, pagA,* or *lef* nucleic acid molecules. Methods of designing primers and probes are disclosed herein, and representative examples of primers and probes that amplify and hybridize to *B. anthracis capB, pagA,* or *lef* nucleic acid molecules are provided.

Articles of manufacture of the invention also can include one or more fluorescent moieties for labeling the probes or, alternatively, the probes supplied with the kit can be labeled. For example, an article of manufacture may include a donor fluorescent moiety for labeling one of the *capB, pagA,* or *lef* probes and an acceptor fluorescent moiety for labeling the other *capB, pagA,* or *lef* probe, respectively. Examples of suitable FRET donor fluorescent moieties and corresponding acceptor fluorescent moieties are provided above.

Articles of manufacture of the invention also can contain a package insert or package label having instructions thereon for using the *capB* primers and probes, *pagA* primers and probes, or *lef* primers and probes to detect *B. anthracis* in a sample. Articles of manufacture may additionally include reagents for carrying out the methods disclosed herein (*e.g.*, buffers, polymerase enzymes, co-factors, or agents to prevent contamination). Such reagents may be specific for one of the commercially available instruments described herein.

The invention will be further described in the following examples, which do not limit the scope of the invention described in the claims.

**EXAMPLES**

Example 1—Oligonucleotide primers and probes

Primers and probes were designed using the OLIGO software (Molecular Biology Insights, Inc., Cascade, OR). Sequences for primers and probes are shown in Table 1. The GenBank Accession numbers for the reference sequences used to design the primers and probes for each target are shown in Table 1, along with the relative location of each primer and probe.

| Gene Target | Gene Bank Accession # | Position (Product Size, bp) | Primer/ Probe Name | Sequence (Base Pair Numbering) |
|---|---|---|---|---|
| Encapsulation Protein B (*capB*) (Plasmid pX02) | M24150 | 371 – 628 (258) | *capB*-F | 5' CCCAATTCGAGTAAACATA 3' (Forward Primer) (371 - 389) |
| | | | *capB*-R | 5' ACTGCCATACATTCACAA 3' (Reverse Primer) (611 - 628) |
| | | | *capB*-FL | 5' CGATTAAGCGCCGTAAAGAAGGTCCTAATATC – FITC 3' (523 – 554) |
| | | | *capB*-RD | 5' Red640 – GTGAGCAACGCAGGGTAGTTAAAGAGGCTG – Phosphate 3' (556 – 585) |
| Protective Antigen (*pagA*) (Plasmid pX01) | M22589 | 2607 – 2935 (329) | *pagA*-F | 5' TACAGGACGGATTGATAAG 3' (Forward Primer) (2607 – 2625) |
| | | | *pagA*-R | 5' TTTCAGCCCAAGTTCTTT 3' (Reverse Primer) (2928 – 2935) |
| | | | *pagA*-FL | 5' AGTACATGGAAATGCAGAAGTG – FITC 3' (2796 – 2817) |
| | | | *pagA*-RD | 5' Red640 – ATGCGTCGTTCTTTGATATTGGT – Phosphate 3' (2819 – 2841) |
| Lethal Factor (*lef*) (Plasmid pX01) | M30210 | 2673 – 3011 (339) | *lef*-F | 5' TTTTACCGATATTACTCTCC 3' (Forward Primer) (2673 – 2692) |
| | | | *lef*-R | 5' AACCTAAAGGCTTCTGC 3' (Reverse Primer) (2995 – 3011) |
| | | | *lef*-FL | 5' ATTAAGGAATGATAGTGAGGGT – FITC 3' (2811 – 2832) |
| | | | *lef*-RD | 5' Red640 – TATACACGAATTTGGACATGCT – Phosphate 3' (2835 – 2856) |

Table 1. Primers and Probes for Detection of *B. anthracis*

Primers *capB*-F and *capB*-R amplified a 258 base pair region. Primers *pagA*-F and *pagA*-R amplified a 329 base pair region. Primers *lef*-F and *lef*-R amplified a 339 base pair region. Primers were adjusted to 50 $^c_cM$ by measuring the $A_{260}$ of a 1/50 dilution (196 µl water + 4 µl, Dilution Factor (DF) = 50). The concentration was estimated by the following formula:

$$(\mu M\ found/50) \times \mu l\ remaining) - \mu l\ remaining = water\ to\ add$$

Probes were dissolved in TE' to a concentration of 20 $^c_cM$ (supplied with the probes and resuspended according to manufacturer's instructions). The concentration of oligonucleotides and dye was double checked by UV absorption using the following equations from *Biochemica,* 1999,1:5-8:

$$[dye] = \frac{A_{dye}}{E_{dye}} \qquad [oligo] = \frac{A_{260} - \left(A_{260} \times \dfrac{E_{260(dye)}}{E_{dye}}\right)}{\dfrac{10^6}{nmol/A_{260}}}$$



| | Absorbance | | | Emission |
|---|---|---|---|---|
| Dye | Abs max | $E_{dye}$ | $E_{260(dye)}$ | Max |
| | (nm) | $(M^{-1}cm^{-1})$ | $(M^{-1}cm^{-1})$ | (nm) |
| Fluorescein | 494 | 68,000 | 2,000 | 524 |
| LC Red 640 | 622 | 110,000 | 31,000 | 638 |

Plasmid controls were produced by cloning the products amplified by the primers for each gene target into a plasmid (TA Clonings® Kit, Invitrogen, Carlsbad, CA). The plasmids (*capB* 371-628/pCR2.1, *pagA* 2607-2935/pCR2.1, *lef* 2673-3011/pCR2.1) containing the target inserts were used to determine the analytical sensitivity of the assays. Plasmid concentration or the copy number of the gene target insert was determined with the following formula: DS DNA, $A_{260}$ to molecules/$^c_c$l

Given:

1.

$$(A_{260} \times \text{Dilution Factor})/20 = \text{mg/ml} = {}^c_cg/{}^c_cl \text{ DS DNA}$$

$1 A_{260} = 50 \, {}^c_cg/ml$
$1 A_{260} (50) = {}^c_cg/ml$
$1 A_{260} (50)/1000 = {}^c_cg/{}^c_cl$

2.

$$(6.02 \times 10^{23} \text{ molecules/mole}) / (10^{12} \text{ pmole/mole}) = 6.02 \times 10^{11} \text{ molecules/pmole}$$

3. Base pairs of DNA in molecule = N

Then:

$$(A_{260} \times DF)/20 \, {}^c_cg/{}^c_cl \times 10^6 \text{ pg}/{}^c_cg \times 1 \text{ pmol/660 pg} \times 1/N \times 6.02 \times 10^{11} \text{ molecules/pmole} = \text{molecules}/{}^c_cl$$

Shortcut calculation:

$$((A_{260} \times DF)/20) \times (9.12 \times 10^{14}/N) = \text{molecules}/{}^c_cl$$

The analytical sensitivities for the three gene targets (*i.e., capB, pagA,* or *lef*) were at least 10 copies of the target sequence. The plasmid controls were used as positive controls for the assay so that infectious *B. anthracis* organisms did not have to be maintained in the laboratory.

Example 2—PCR Conditions

The LightCycler™ Hybridization Mix was identical for each *B. anthracis* gene target (with the exception that each primer and probe set was specific for the particular gene target that was amplified).

| LightCycler™ Hybridization Mix - *B. anthracis capB, pagA* or *lef* | | | |
|---|---|---|---|
| Ingredient | Stock | Final | $^c$cl |
| Water | -- | -- | 863 |
| MgCl$_2$ | 50 mM | 5 mM | 160 |
| 10X buffer | 10 X | 1 X | 200 |
| Primer-F | 50 mM | 0.5 mM | 20 |

(continued)

| LightCycler™ Hybridization Mix - *B. anthracis capB, pagA* or *lef* | | | |
|---|---|---|---|
| Ingredient | Stock | Final | $^c$cl |
| Primer-R | 50 mM | 0.5 mM | 20 |
| Platinum® Taq | 5 U/ml | 0.030 U/ml | 12 |
| dNTP Plus | 10 mM | 0.2 mM | 40 |
| BSA | 2% | 0.025% | 25 |
| HK-UNG | 10% | 0.2% | 40 |
| Probe-FL | 10 mM | 0.2 mM | 40 |
| Probe-RD | 10 mM | 0.2 mM | 40 |
| Total volume | | | 1500 |

The LightCycler™ thermocycling conditions also were the same for each gene target.
Fluorescence Settings:

| LED Power | CALIB |
|---|---|
| F1 Gain | 1 |
| F2 Gain | 15 |

Quantification Settings:

| Channel Settings | F2/F1 |
|---|---|
| Name of Program | |

Experimental Protocol:

Uracil-DNA glycosylase (1 cycle) Type: none
 37°C, 300 sec, 20°/sec slope
 95°C, 180 sec, 20°/sec slope
PCR (45 cycles) Type: Quantification
 95°C, 0 sec hold, 20°C/sec slope
 55°C, 15 sec hold, 20°C/sec slope, single acquisition
 72°C, 12 sec hold, 20°C/sec slope
Melt (1 cycle) Type: Melting Curve
 95°C, 0 sec hold, 20°C/sec slope
 50°C, 30 sec hold, 2°C/sec slope
 85°C, 0 sec hold, 0.2°C/sec slope, continuous acquisition
Cool (1 cycle) Type: None
 40°, 30 sec hold, 20°C/sec slope

Example 3—Melting Curves

Following the completion of the amplification reactions, a melting curve analysis was performed by raising the temperature in the LightCycler™ thermal chamber from 50°C to 85°C at 0.2°C per second. Fluorescent measurements were taken continuously as the temperature was raised and melting curves were generated. Each pair of probes had a specific and characteristic melting curve from the respective amplification product. The melting temperatures (Tm) for the products generated were 70.4 ± 1°C for *capB,* 64.2 ± 1°C for *pagA*, and 58.4 ± 1°C for *lef*.

Example 4—Analytical Sensitivity

A total of 32 isolates of *B. anthracis* were studied including strains used for vaccine preparation. Twenty-eight isolates were positive for each of the three gene targets. *B. anthracis* 'delta Ames' which lacks plasmid pX01 was only positive for *capB*. *B. anthracis* 'Sterne' lacks plasmid pX02 and was positive for *pagA* and *lef*, but not for *capB.* There was a single isolate (GT 25) that produced a false negative for *capB.*

The following *B. anthracis* strains were used. With the exception of the 'Mayo' strains, all others were provided by Dr. Ted Hadfield (Armed Forces Institute of Pathology, Washington, D.C.): *B. anthracis* 'Ames'; *B. anthracis* 'delta-Ames' (lacks plasmid pX01); *B. anthracis* 'GT 3'; *B. anthracis* 'GT 10'; *B. anthracis* 'GT 15'; *B. anthracis* 'GT 20'; *B. anthracis* 'GT 23'; *B. anthracis* 'GT 25'; *B. anthracis* 'GT 28'; *B. anthracis* 'GT 29'; *B. anthracis* 'GT 30'; *B. anthracis* 'GT 34'; *B. anthracis* 'GT 35'; *B. anthracis* 'GT 38'; *B. anthracis* 'GT 41'; *B. anthracis* 'GT 45'; *B. anthracis* 'GT 51'; *B. anthracis* 'GT 55'; *B. anthracis* 'GT 57'; *B. anthracis* 'GT 62'; *B. anthracis* 'GT 68'; *B. anthracis* 'GT 69'; *B. anthracis* 'GT 77'; *B. anthracis* 'GT 80'; *B. anthracis* 'GT 85'; *B. anthracis* 'GT 87'; *B. anthracis* 'Mayo 1'; *B. anthracis* 'Mayo 2'; *B. anthracis* 'Mayo 3'; *B. anthracis* 'Sterne' (lacks plasmid pX02); *B. anthracis* 'Vollum'; and *B. anthracis* 'Zimbabwe'.

Example 5—Analytical Specificity

The following organisms were tested as described above in Examples 2 and 3 for cross reactivity with the primers and probes directed toward *B. anthracis.* There was no cross-reactivity observed. Some of the organisms listed below were obtained from Mayo clinical isolates; others were ATCC reference strains.

The following strains were used: *B. amyloliquefaciens* (1 strain); *B. cereus* (4 clinical, 8 strains); *B. globigii* (1 strain); *B. halodurans* (2 clinical); *B. megaterium* (2 clinical, 1 strain); *B. mycoides* (1 strain); *B. sphaericus* (1 strain); *B. subtilis* (1 clinical, 1 strain); *B. thuringiensis* (3 strains); *B. thuringiensis israeliensis* (1 strain); *B. thuringiensis kurstaki* (1 strain); *Bacteriodes fragilis* (1 strain); *Brucella abortus* (1 strain); *Brucella maris* (1 strain); *Brucella ovis* (1 strain); *Burkholderia cocovenans* (1 strain); *Clostridium perfringens* (2 strains); *Clostridium sporogenes* (1 strain); *Corynebacterium diphtheriae* (1 strain); *Corynebacterium jeikeium* (1 strain); Deinococcus *radiodurans* (1 strain); *Dichleobacter nodosus* (1 strain); *Francisella tularensis* (1 strain); *Listeria innocua* (1 strain); *Paenibacillus pabulii* (2 clinical); *Peptostreptococcus anaerobius* (1 strain); *Pseudomonas stutzeri* (1 strain); *Staphylococcus aureus* (1 strain); *Vibrio cholerae* (1 strain); *Yersinia ruckeri* (1 strain).

**OTHER EMBODIMENTS**

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

**Claims**

1. A method for detecting the presence or absence of *B. anthracis* in a biological sample from an individual or in a non-biological sample, said method comprising:

performing at least one cycling step, wherein a cycling step comprises an amplifying step and a hybridizing step, wherein said amplifying step comprises contacting said sample with a pair of *capB* primers to produce a *capB* amplification product if a *B. anthracis capB* nucleic acid molecule is present in said sample, wherein said hybridizing step comprises contacting said sample with a pair of *capB* probes, wherein the members of said pair of *capB* probes hybridize within no more than five nucleotides of each other, wherein a first *capB* probe of said pair of *capB* probes is labeled with a donor fluorescent moiety and said second *capB* probe of said pair of *capB* probes is labeled with a corresponding acceptor fluorescent moiety; and

detecting the presence or absence of fluorescence resonance energy transfer (FRET) between said donor fluorescent moiety of said first *capB* probe and said acceptor fluorescent moiety of said second *capB* probe,

wherein the presence of FRET is indicative of the presence of *B. anthracis* in said sample, and wherein the absence of FRET is indicative of the absence of *B. anthracis* in said sample.

**2.** The method of claim 1, further comprising:

performing at least one cycling step, wherein said cycling step comprises an amplifying step and a hybridizing step, wherein said amplifying step comprises contacting said sample with a pair of *pagA* primers to produce a *pagA* amplification product if a *B. anthracis pagA* nucleic acid molecule is present in said sample, wherein said hybridizing step comprises contacting said sample with a pair of *pagA* probes, wherein the members of said pair of *pagA* probes hybridize within no more than five nucleotides of each other, wherein a first *pagA* probe of said pair of *pagA* probes is labeled with a donor fluorescent moiety and said second *pagA* probe of said pair of *pagA* probes is labeled with a corresponding acceptor fluorescent moiety; and

detecting the presence or absence of FRET between said donor fluorescent moiety of said first *pagA* probe and said acceptor fluorescent moiety of said second *pagA* probe.

**3.** The method of claim 2, further comprising:

performing at least one cycling step, wherein said cycling step comprises an amplifying step and a hybridizing step, wherein said amplifying step comprises contacting said sample with a pair of *lef* primers to product a *lef* amplification product if a *B. anthracis lef* nucleic acid molecule is present in said sample, wherein said hybridizing step comprises contacting said sample with a pair of *lef* probes, wherein the members of said pair of *lef* probes hybridize within no more than five nucleotides of each other, wherein a first *lef* probe of said pair of *lef* probes is labeled with a donor fluorescent moiety and said second *lef* probe of said pair of *lef* probes is labeled with a corresponding acceptor fluorescent moiety; and

detecting the presence or absence of FRET between said donor fluorescent moiety of said first *lef* probe and said acceptor fluorescent moiety of said second *lef* probe.

**4.** A method for detecting the presence or absence of *B. anthracis* in a biological sample from an individual or in a non-biological sample, said method comprising:

performing at least one cycling step, wherein a cycling step comprises an amplifying step and a hybridizing step, wherein said amplifying step comprises contacting said sample with a pair of *pagA* primers to produce a *pagA* amplification product if a *B. anthracis pagA* nucleic acid molecule is present in said sample, wherein said hybridizing step comprises contacting said sample with a pair of *pagA* probes, wherein the members of said pair of *pagA* probes hybridize within no more than five nucleotides of each other, wherein a first *pagA* probe of said pair of *pagA* probes is labeled with a donor fluorescent moiety and said second *pagA* probe of said pair of *pagA* probes is labeled with a corresponding acceptor fluorescent moiety; and

detecting the presence or absence of fluorescence resonance energy transfer (FRET) between said donor fluorescent moiety of said first *pagA* probe and said acceptor fluorescent moiety of said second *pagA* probe,

wherein the presence of FRET is indicative of the presence of *B. anthracis* in said sample, and wherein the absence of FRET is indicative of the absence of *B. anthracis* in said sample.

**5.** A method for detecting the presence or absence of *B. anthracis* in a biological sample from an individual or in a non-biological sample, said method comprising:

performing at least one cycling step, wherein a cycling step comprises an amplifying step and a hybridizing step, wherein said amplifying step comprises contacting said sample with a pair of *lef* primers to produce a *lef* amplification product if a *B. anthracis lef* nucleic acid molecule is present in said sample, wherein said hybridizing step comprises contacting said sample with a pair of *lef* probes, wherein the members of said pair of *lef* probes hybridize within no more than five nucleotides of each other, wherein a first *lef* probe of said pair of *lef* probes is labeled with a donor fluorescent moiety and said second *lef* probe of said pair of *lef* probes is labeled with a corresponding acceptor fluorescent moiety; and

detecting the presence or absence of fluorescence resonance energy transfer (FRET) between said donor fluorescent moiety of said first *lef* probe and said acceptor fluorescent moiety of said second *lef* probe,

wherein the presence of FRET is indicative of the presence of *B. anthracis* in said sample, and wherein the absence of FRET is indicative of the absence of *B. anthracis* in said sample.

6. An article of manufacture, comprising:

a pair of *capB* primers;
a pair of *capB* probes; and
a donor fluorescent moiety and a corresponding acceptor fluorescent moiety.

7. An article of manufacture, comprising:

a pair of *pagA* primers;
a pair of *pagA* probes; and
a donor fluorescent moiety and a corresponding acceptor fluorescent moiety.

8. An article of manufacture, comprising:

a pair of *lef* primers;
a pair of *lef* probes; and
a donor fluorescent moiety and a corresponding acceptor fluorescent moiety.

9. A method for detecting the presence or absence of *B. anthracis* in a biological sample from an individual or in a non-biological sample, said method comprising:

performing at least one cycling step, wherein a cycling step comprises an amplifying step and a hybridizing step, wherein said amplifying step comprises contacting said sample with a pair of *capB* primers to produce a *capB* amplification product if a *B. anthracis capB* nucleic acid molecule is present in said sample, wherein said hybridizing step comprises contacting said sample with a *capB* probe, wherein the *capB* probe is labeled with a donor fluorescent moiety and a corresponding acceptor fluorescent moiety; and

detecting the presence or absence of fluorescence resonance energy transfer (FRET) between said donor fluorescent moiety and said acceptor fluorescent moiety of said *capB* probe,

wherein the presence or absence of fluorescence is indicative of the presence or absence of *B. anthracis* in said sample.

10. A method for detecting the presence or absence of *B. anthracis* in a biological sample from an individual or in a non-biological sample, said method comprising:

performing at least one cycling step, wherein a cycling step comprises an amplifying step and a dye-binding step, wherein said amplifying step comprises contacting said sample with a pair of *capB* primers to produce a *capB* amplification product if a *B. anthracis capB* nucleic acid molecule is present in said sample, wherein said dye-binding step comprises contacting said *capB* amplification product with a double-stranded DNA binding dye; and

detecting the presence or absence of binding of said double-stranded DNA binding dye into said amplification product,

wherein the presence of binding is indicative of the presence of *B. anthracis* in said sample, and wherein the absence of binding is indicative of the absence of *B. anthracis* in said sample.

European Patent

Office

**PARTIAL EUROPEAN SEARCH REPORT**

Application Number

which under Rule 45 of the European Patent Convention EP 02 02 2398
shall be considered, for the purposes of subsequent
proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | BELL C A ET AL: "Direct detection of Bacillus anthracis using a real-time PCR method." ABSTRACTS OF THE GENERAL MEETING OF THE AMERICAN SOCIETY FOR MICROBIOLOGY, vol. 101, 2001, page 155 XP002226384 101st General Meeting of the American Society for Microbiology;Orlando, FL, USA; May 20-24, 2001, http://www.asmusa.org/mtgsrc/generalmeeting.htm 2001 ISSN: 1060-2011 | 1 | C12Q1/68 |
| Y | * the whole document * | 2-10 | |

-/--

TECHNICAL FIELDS
SEARCHED     (Int.Cl.7)

C12Q

## INCOMPLETE SEARCH

The Search Division considers that the present application, or one or more of its claims, does/do
not comply with the EPC to such an extent that a meaningful search into the state of the art cannot
be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| MUNICH | 10 January 2003 | Schmitt, C |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

European Patent
Office

INCOMPLETE SEARCH
SHEET C

Application Number

EP 02 02 2398

Claim(s) searched incompletely:
    1-10

Reason for the limitation of the search:

1) Claim 6 relates to an article of manufacture comprising a pair of capB primers, a pair of capB probes, and a donor fluorescent moiety and a corresponding acceptor fluorescent moiety. However, these sole features are not sufficient to characterize said primers, probes and fluorescent moieties so as to allow the skilled person to clearly and unambiguously understand the scope of said claim (Article 84 EPC, see also Guidelines C.II.6.3.i).
Furthermore, claim 6 relates to an extremely large number of possible capB oligonucleotides suitable as primers or probes. Additionally, claim 6 relates to an extremely large number of possible fluorescent moieties (e.g. proteins, antibodies, oligonucleotides, nucleic acids, fluorescein, Cy5).
The search was therefore limited to oligonucleotides, suitable as primers or probes, which are encompassed by the sequence corresponding to nucleotides 282 to 1475 of the sequence of Fig. 3 disclosed in Makino et al. (Journal of Bacteriology, 1989, 171(2), pp 722-730) and specifically to capB primers having SEQ ID Nos 1-2 and to capB probes having SEQ ID Nos: 3, 4, 13 and 14. Additionally, the search was also limited to donor and acceptor fluorophore molecules suitable for fluorescent resonance energy transfer (FRET).

2) The same objections apply to claims 7 and 8.
The search was, therefore, limited to oligonucleotides of the pagA gene, suitable as primers or probes, which are encompassed by the sequence corresponding to nucleotides 1804 to 4098 of the sequence of Fig. 2 disclosed in Welkos et al. (Gene, 1988, 69, pp 287-300) and specifically to pagA primers having SEQ ID Nos: 5-6 and to pagA probes having SEQ ID Nos: 7, 8, 15 and 16.
Furthermore, the search was also limited to oligonucleotides of the lef gene, suitable as primers or probes, which are encompassed by the sequence corresponding to nucleotides 481 to 2910 of the sequence of Fig.2 disclosed in Bragg and Robertson (Gene, 1989, 81, pp 45-54) and specifically to lef primers having SEQ ID Nos: 9-10 and to lef probes having SEQ ID Nos: 11 and 12. Additionally, the search was also limited to donor and acceptor fluorophore molecules suitable for FRET.

3) Claims 1, 9 and 10 relate to methods for detecting B. anthracis in a sample, said methods comprising an amplifying step and a hybridizing step, wherein said amplifying and hybridizing steps comprise contacting said sample with a pair of capB primers and with a pair of capB probes, respectively. Taking into account the objections put forward in item 1), the search was, therefore, limited as for claim 6.

The same objections apply to claims 2 and 3, which are dependent on independent claim 1, 4 and 5. The search of claims 2 and 3 was, therefore, limited as for claim 7 and 8, respectively.

European Patent Office

**PARTIAL EUROPEAN SEARCH REPORT**

Application Number

EP 02 02 2398

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| X | RAMISSE V ET AL: "Identification and characterization of Bacillus anthracis by multiplex PCR analysis of sequences on plasmids pX01 and pX02 and chromosomal DNA." FEMS MICROBIOLOGY LETTERS. NETHERLANDS 15 NOV 1996, vol. 145, no. 1, 15 November 1996 (1996-11-15), pages 9-16, XP002226327 ISSN: 0378-1097 * page 9, column 1 - page 22, column 2, paragraph 2 * * page 12, column 2, paragraph 1 * * table 2 * * figure 1 * | 10 | |
| Y | * page 13, column 2, paragraph 2 - page 15, column 1, paragraph 1 * | 1-9 | |
| X | REIF T C ET AL: "Identification of capsule-forming Bacillus anthracis spores with the PCR and a novel dual-probe hybridization format." APPLIED AND ENVIRONMENTAL MICROBIOLOGY. UNITED STATES MAY 1994, vol. 60, no. 5, May 1994 (1994-05), pages 1622-1625, XP009003142 ISSN: 0099-2240 *abstract* * page 1623, column 1, paragraph 4 * | 10 | |
| Y | * page 1623, column 1, paragraph 2 - column 2, paragraph 1 * | 1,6,9 | |

TECHNICAL FIELDS SEARCHED (Int.Cl.7)

-/--

**European Patent Office**

## PARTIAL EUROPEAN SEARCH REPORT

Application Number

EP 02 02 2398

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| Y | QI Y ET AL: "Utilization of the rpoB gene as a specific chromosomal marker for real-time PCR detection of Bacillus anthracis." APPLIED AND ENVIRONMENTAL MICROBIOLOGY. UNITED STATES AUG 2001, vol. 67, no. 8, August 2001 (2001-08), pages 3720-3727, XP002226328 ISSN: 0099-2240 * abstract * * page 3720, column 2, paragraph 2 * * page 3724, column 1, paragraph 3 - column 2, paragraph 2 * * page 3726, column 2, paragraph 3 * * figure 1 * | 1-10 | |
| Y | MAKINO S I ET AL: "Detection of anthrax spores from the air by real-time PCR." LETTERS IN APPLIED MICROBIOLOGY. ENGLAND SEP 2001, vol. 33, no. 3, September 2001 (2001-09), pages 237-240, XP002226329 ISSN: 0266-8254 * page 238, column 1, paragraph 1 - page 239, column 2, paragraph 2 * * figure 3 *  -/-- | 1-10 | TECHNICAL FIELDS SEARCHED (Int.Cl.7) |

EPO FORM 1503 03.82 (P04C10)

EP 1 304 387 A1

**European Patent Office**

**PARTIAL EUROPEAN SEARCH REPORT**

Application Number

EP 02 02 2398

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | LEE M A ET AL: "Fluorescent detection techniques for real-time multiplex strand specific detection of Bacillus anthracis using rapid PCR." JOURNAL OF APPLIED MICROBIOLOGY. ENGLAND AUG 1999, vol. 87, no. 2, August 1999 (1999-08), pages 218-223, XP002226330 ISSN: 1364-5072 * page 218, column 2, paragraph 1 * * page 219, column 1, paragraph 3 * * page 220, column 2, paragraph 4 - page 222, column 1, paragraph 1 * | 1-10 | |
| Y | WITTWER C T ET AL: "CONTINUOUS FLUORESCENCE MONITORING OF RAPID CYCLE DNA AMPLIFICATION" BIOTECHNIQUES, EATON PUBLISHING, NATICK, US, vol. 22, no. 1, 1997, pages 130-131,134-138, XP000683698 ISSN: 0736-6205 * the whole document * | 1,4,5,9, 10 | **TECHNICAL FIELDS SEARCHED** (Int.Cl.7) |
| Y | MAKINO S ET AL: "Molecular characterization and protein analysis of the cap region, which is essential for encapsulation in Bacillus anthracis." JOURNAL OF BACTERIOLOGY. UNITED STATES FEB 1989, vol. 171, no. 2, February 1989 (1989-02), pages 722-730, XP009003455 ISSN: 0021-9193 *Fig.3, nucleotides 282 to 1475* | 1,6,9,10 | |

-/--

**European Patent**
**Office**

**PARTIAL EUROPEAN SEARCH REPORT**

Application Number

EP 02 02 2398

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| Y | BRAGG T S ET AL: "Nucleotide sequence and analysis of the lethal factor gene (lef) from Bacillus anthracis." GENE. NETHERLANDS 1 SEP 1989, vol. 81, no. 1, 1 September 1989 (1989-09-01), pages 45-54, XP002942518 ISSN: 0378-1119 *Fig. 2, nucleotides 481 to 2910* | 1,3,5,8 | |
| Y | WELKOS S L ET AL: "SEQUENCE AND ANALYSIS OF THE DNA ENCODING PROTECTIVE ANTIGEN OF BACILLUS-ANTHRACIS" GENE, ELSEVIER BIOMEDICAL PRESS. AMSTERDAM, NL, vol. 69, no. 2, 1988, pages 287-300, XP002183269 ISSN: 0378-1119 *Fig. 2, nucleotides 1804 to 4098* | 1,2,4,7 | TECHNICAL FIELDS SEARCHED (Int.Cl.7) |
| A | MAKINO S I ET AL: "Direct detection of Bacillus anthracis DNA in animals by polymerase chain reaction." JOURNAL OF CLINICAL MICROBIOLOGY. UNITED STATES MAR 1993, vol. 31, no. 3, March 1993 (1993-03), pages 547-551, XP009003297 ISSN: 0095-1137 * the whole document * | 1-5,9,10 | |
| A | ROCHE MOLECULAR BIOCHEMICALS: "LightCycler Operator's Manual, Version 3.5" ROCHE MOLECULAR BIOCHEMICALS, October 2000 (2000-10), pages 97-160, XP002226589 * page 103 - page 109 * * page 111 - page 114 * | 1-5,9,10 | |

-/--

## PARTIAL EUROPEAN SEARCH REPORT

European Patent Office

Application Number

EP 02 02 2398

### DOCUMENTS CONSIDERED TO BE RELEVANT

| Category | Citation of document with indication, where appropriate, of relevant passages | Relevant to claim |
|---|---|---|
| P,X | BELL CONSTANCE A ET AL: "Detection of Bacillus anthracis DNA by LightCycler PCR." JOURNAL OF CLINICAL MICROBIOLOGY. UNITED STATES AUG 2002, vol. 40, no. 8, August 2002 (2002-08), pages 2897-2902, XP009003109 ISSN: 0095-1137 *abstract* * page 2898, column 2, paragraph 1 - page 2899, column 2, paragraph 2 * | 1-9 |
| P,Y | * page 2898, column 1, paragraph 2 * | 10 |

CLASSIFICATION OF THE APPLICATION (Int.Cl.7)

TECHNICAL FIELDS SEARCHED (Int.Cl.7)

EPO FORM 1503 03.82 (P04C10)

EP 1 304 387 A1

**European Patent**
Office

Application Number

EP 02 02 2398

---

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

---

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☒ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

**EP 1 304 387 A1**

<table>
<tr><td>European Patent<br>Office</td><td>**LACK OF UNITY OF INVENTION**<br>**SHEET B**</td><td>Application Number<br>EP 02 02 2398</td></tr>
</table>

The Search Division considers that the present European patent application does not comply with the requirements of unity of invention and relates to several inventions or groups of inventions, namely:

1. Claims: 1, 6, 9, 10 (completely), 2, 3 (partially)

    Methods for detecting Bacillus anthracis using primers and probes derived from the capB genes and article of manufacture containing such primers and probes.

2. Claims: 4, 7 (completely), 2, 3 (partially)

    Methods for detecting Bacillus anthracis using primers and probes derived from the pagA gene and article of manufacture containing such primers and probes.

3. Claims: 5, 8 (completely), 3 (partially)

    Methods for detecting Bacillus anthracis using primers and probes derived from the lef gene and article of manufacture containing such primers and probes.

SEQUENCE LISTING

<110> Bell, Constance A.
      Uhl, James
      Cockerill, Franklin

<120> Detection of Bacillus Anthracis

<130> 07039-372001

<140> US 10/068,238
<141> 2002-02-05

<150> US 60/329,826
<151> 2001-10-15

<160> 16

<170> FastSEQ for Windows Version 4.0

<210> 1
<211> 19
<212> DNA
<213> Artificial Sequence
<220>
<223> Oligonucleotide
<400> 1
cccaattcga gtaaacata                                                    19

<210> 2
<211> 18
<212> DNA
<213> Artificial Sequence
<220>
<223> Oligonucleotide
<400> 2
actgccatac attcacaa                                                     18

<210> 3
<211> 32
<212> DNA
<213> Artificial Sequence
<220>
<223> Oligonucleotide
<400> 3
cgattaagcg ccgtaaagaa ggtcctaata tc                                     32

<210> 4
<211> 30
<212> DNA
<213> Artificial Sequence
<220>
<223> Oligonucleotide
<400> 4
gtgagcaacg cagggtagtt aaagaggctg                                        30

```
<210> 5
<211> 19
<212> DNA
<213> Artificial Sequence
<220>
<223> Oligonucleotide
<400> 5
tacaggacgg attgataag                                                19


<210> 6
<211> 18
<212> DNA
<213> Artificial Sequence
<220>
<223> Oligonucleotide
<400> 6
tttcagccca agttcttt                                                 18


<210> 7
<211> 22
<212> DNA
<213> Artificial Sequence
<220>
<223> Oligonucleotide
<400> 7
agtacatgga aatgcagaag tg                                            22


<210> 8
<211> 23
<212> DNA
<213> Artificial Sequence
<220>
<223> Oligonucleotide
<400> 8
atgcgtcgtt ctttgatatt ggt                                           23


<210> 9
<211> 20
<212> DNA
<213> Artificial Sequence
<220>
<223> Oligonucleotide
<400> 9
ttttaccgat attactctcc                                               20


<210> 10
<211> 17
<212> DNA
<213> Artificial Sequence
<220>
<223> Oligonucleotide
<400> 10
aacctaaagg cttctgc                                                  17
```

```
<210> 11
<211> 22
<212> DNA
<213> Artificial Sequence
<220>
<223> Oligonucleotide
<400> 11
attaaggaat gatagtgagg gt                               22


<210> 12
<211> 22
<212> DNA
<213> Artificial Sequence
<220>
<223> Oligonucleotide
<400> 12
tatacacgaa tttggacatg ct                               22


<210> 13
<211> 25
<212> DNA
<213> Artificial Sequence
<220>
<223> Oligonucleotide
<400> 13
cgccgtaaag aaggtcctaa tatcg                            25


<210> 14
<211> 26
<212> DNA
<213> Artificial Sequence
<220>
<223> Oligonucleotide
<400> 14
tgagcaacgc agggtagtta aagagg                           26


<210> 15
<211> 25
<212> DNA
<213> Artificial Sequence
<220>
<223> Oligonucleotide
<400> 15
gtgcatgcgt cgttctttca tattg                            25


<210> 16
<211> 30
<212> DNA
<213> Artificial Sequence
<220>
<223> Oligonucleotide
<400> 16
tgggagtgta tctgcaggat ttagtaattc                       30
```